(19) Europäisches Patentamt
European Patent Office
Office européen des brevets

(11) EP 1 004 575 B1

(12) # EUROPÄISCHE PATENTSCHRIFT

(45) Veröffentlichungstag und Bekanntmachung des Hinweises auf die Patenterteilung:
**04.06.2003 Patentblatt 2003/23**

(51) Int Cl.$^7$: **C07C 275/70**, C07C 273/00

(21) Anmeldenummer: **99123307.3**

(22) Anmeldetag: **22.11.1999**

(54) **Verfahren zur Herstellung von O-Alkylisoharnstoff**

Process for the preparation of O-alkylisourea

Procédé pour la préparation de O-alkylisourée

(84) Benannte Vertragsstaaten:
**AT BE CH CY DE DK ES FI FR GB GR IE IT LI LU MC NL PT SE**
Benannte Erstreckungsstaaten:
**RO SI**

(30) Priorität: **23.11.1998 DE 19853984**

(43) Veröffentlichungstag der Anmeldung:
**31.05.2000 Patentblatt 2000/22**

(73) Patentinhaber: **BASF AKTIENGESELLSCHAFT 67056 Ludwigshafen (DE)**

(72) Erfinder:
- **Kluge, Michael 67071 Ludwigshafen (DE)**
- **Kessel, Knut 68161 Mannheim (DE)**
- **Greindl, Thomas 67098 Bad Dürkheim (DE)**
- **Biedermann, Norbert 67098 Bad Dürkheim (DE)**
- **Scherr, Günter 67065 Ludwigshafen (DE)**
- **Bogenstätter, Thomas 67098 Bad Dürkheim (DE)**

(74) Vertreter: **Kinzebach, Werner, Dr. et al Ludwigsplatz 4 67059 Ludwigshafen (DE)**

(56) Entgegenhaltungen:
- **J. KRECHL ET AL: "Simple amidinium carboxylates - an MO treatment of molecular geometry and electronic structure" COLLECTION OF CZECHOSLOVAK CHEMICAL COMMUNICATIONS., Bd. 54, 1989, Seiten 673-683, XP002132935 Prague ISSN: 0010-0765**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 004 575 B1

## Beschreibung

Die vorliegende Erfindung betrifft ein Verfahren zur Herstellung von O-Alkylisoharnstoffen in Form ihrer Säureadditionssalze.

O-Alkylisoharnstoffe bzw. ihre Säureadditionssalze sind wertvolle Zwischenprodukte, die beispielsweise mit primären oder sekundären Aminen zu substituierten Guanidiniumverbindungen umgesetzt werden können. Substituierte Guanidiniumverbindungen sind in der Natur weit verbreitet. Wichtige Vertreter dieser Substanzklasse sind beispielsweise Aminosäuren, wie Arginin und Kreatin. Darüber hinaus sind substituierte Guanidinverbindungen als sterisch gehinderte Basen, als Biozide sowie als Komplexliganden bekannt. Die Mehrzahl der Verbindungen dieses Typs sind aufgrund der hohen Herstellungskosten in ihrer technischen Anwendbarkeit jedoch stark eingeschränkt.

Eine Möglichkeit zur Herstellung von O-Alkylisoharnstoffen besteht in der Umsetzung von Harnstoff mit Alkylgruppen-übertragenden Reagenzien, wie Dialkylsulfaten, vgl. z. B. Collect. Czech. Chem. Commun., 1989, 54, S. 678. Die Synthese von O-Methylisoharnstoff durch Alkylierung von Harnstoff mit Dimethylsulfat ist z.B. möglich, indem der Harnstoff im Dialkylsulfat suspendiert und das Gemisch erwärmt wird. Die Umsetzung verläuft stark exotherm. Einen Nachteil stellt dabei die schlechte Löslichkeit des Harnstoffs im Dialkylsulfat dar. Die Umsetzung springt daher nur mit Verzögerung an. Mit dem Fortschreiten der Reaktion wird aber immer mehr Harnstoff nachgelöst, was dazu führt, dass die Reaktion rasch außer Kontrolle geraten kann. Dies stellt unter Sicherheitsaspekten ein ernstes Problem dar. Als Folge einer unkontrollierten Reaktion werden unerwünschte N-Alkylierungen und Mehrfachalkylierungen beobachtet. Außerdem sind O-Alkylisoharnstoffe oberhalb von Temperaturen von 100°C thermisch zersetzlich. Durch die genannten Umstände werden die Reinheit und die Ausbeute des Produkts beeinträchtigt.

Die JP 62-030983 empfiehlt die Umsetzung von Harnstoff und Dimethylsulfat in Gegenwart von 7 bis 30 ml Methanol pro mol Harnstoff bzw. Dimethylsulfat durchzuführen. Auf diese Weise soll eine Siedekühlung erreicht werden, d.h. die Temperaturerhöhung bei der exothermen Reaktion wird begrenzt, weil die Reaktionswärme teilweise durch die Verdampfungswärme des Methanols aufgezehrt wird. Nachteilig ist allerdings bei diesem Verfahren, dass in einer Nebenreaktion aus Methanol und Dimethylsulfat Dimethylether gebildet wird. Dies ist wegen der Explosionsgefahr des Dimethylethers und des Verlustes an Alkylierungsmittel unerwünscht. Die Bildung von Dimethylether bzw. die Verdampfung des Methanols führen außerdem zu einem starken Druck- bzw. Volumenanstieg, der die großtechnische Durchführung der beschriebenen Umsetzung erschwert.

Der vorliegenden Erfindung liegt daher die Aufgabe zugrunde, ein kostengünstiges und einfach durchzuführendes Verfahren zur Herstellung von O-Alkylisoharnstoffen in Form ihrer Säureadditionssalze bereitzustellen, das eine einfache Reaktionsführung erlaubt und in hoher Ausbeute zu einem besonders reinen Produkt führt.

Es wurde überraschenderweise gefunden, dass diese Aufgabe durch eine kontinuierliche Umsetzung von Harnstoff mit einem Alkylgruppen-übertragenden Reagenz gelöst werden kann.

Gegenstand der Erfindung ist daher ein Verfahren zur Herstellung von O-Alkylisoharnstoff der Formel I,

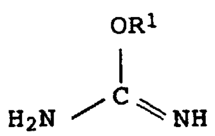

I

worin $R^1$ für $C_1$- bis $C_{20}$-Alkyl steht, in Form eines Säureadditionssalzes, bei dem Harnstoff und ein unter Alkylhalogeniden und Dialkylsulfaten ausgewähltes Alkylgruppen-übertragendes Reagenz, gelöst oder suspendiert in einem Verdünnungsmittel, in einem kontinuierlich betriebenen Reaktor bei einer Temperatur von 40-200°C umgesetzt werden, wobei das Verdünnungsmittel ein O-Alkylisoharnstoff-säureadditionssalz ist.

Der O-Alkylisoharnstoff wird in Form eines Säureadditionssalzes erhalten, in dem der Isoharnstoff in protonierter Form zusammen mit einem Anion vorliegt, das aus dem Alkylgruppen-übertragenden Reagenz stammt. Das Anion kann z.B. ein Halogenid, wie Chlorid, Bromid oder Iodid, Sulfat oder ein $C_1$-$C_{20}$-Alkylsulfat, wie Methylsulfat, sein. Das O-Alkylisoharnstoff-Säureadditionssalz fällt in der Regel als ölige Substanz an. Die O-Alkylisoharnstoff-Säureadditionssalze haben oft Schmelzpunkte von nahe bei oder unterhalb der Handhabungstemperatur, oder ihre Kristallisation ist kinetisch gehemmt. Gewünschtenfalls kann daraus der freie O-Alkylisoharnstoff nach üblichen Verfahren isoliert werden.

Erfindungsgemäß werden Harnstoff und Alkylgruppen-übertragendes Reagenz in einem kontinuierlichen Reaktor bei einer Temperatur von 40-200°C, vorzugsweise 60-120°C, umgesetzt. Als kontinuierlicher Reaktor sind alle gängigen Reaktortypen wie beispielsweise ein kontinuierlicher Rohrreaktor, kontinuierlicher Rührreaktor, oder eine kontinuierliche Rührkesselkaskade geeignet.

Die Umsetzung von Harnstoff mit Alkylgruppen-übertragenden Reagenzien erfordert zunächst eine Energiezufuhr, um die Aktivierungsenergie der Reaktion aufzubringen. Sobald die Reaktion anspringt, wird die exotherme

Reaktionsenthalpie frei. Andererseits treten bei höheren Temperaturen zunehmend Zersetzungsreaktionen und Nebenreaktionen in den Vordergrund. Die erfindungsgemäße kontinuierliche Reaktionsführung gestattet auf überraschend einfache und elegante Weise, diese Problematik zu beherrschen, da im Gegensatz zum diskontinuierlichen Betrieb pro Zeiteinheit nur eine vergleichsweise geringe Menge des Gemisches von Harnstoff und Alkylgruppen-übertragendem Reagenz auf die Reaktionstemperatur gebracht wird. Die Reaktionswärme, die bei der Umsetzung dieser geringen Menge des Gemisches pro Zeiteinheit frei wird, kann andererseits rasch abgeführt oder vom mitverwendeten Verdünnungsmittel absorbiert werden. Es treten somit keine temporären Spitzen zu übertragender Wärmemengen auf, sondern zeitlich im Wesentlichen konstante Wärmeströme.

Das erfindungsgemäße Verfahren wird vorzugsweise in einem Rohrreaktor durchgeführt. Bei einem Rohrreaktor handelt es sich um ein Strömungsrohr, dessen Querschnitt klein gegenüber der Länge ist. Die Form des Querschnitts, z.B. rund oder rechteckig, ist unkritisch für das erfindungsgemäße Verfahren. Der Betrieb des Rohrreaktors erfolgt vorzugsweise auf solche Weise, dass ein enges Verweilzeitspektrum des Reaktionsgemisches erhalten wird. Die einzelnen Stadien der fortschreitenden Umsetzung treten dabei örtlich hintereinander auf.

Bei Verwendung eines Rohrreaktors wird dem eintretenden Gemisch von Harnstoff und Alkylgruppen-übertragendem Reagenz beispielsweise in einem ersten Segment des Rohrreaktors Energie zugeführt, bis die Reaktion anspringt. Das Gemisch erfährt kurzzeitig eine adiabatische Temperaturerhöhung, und in einem zweiten Segment des Rohrreaktors findet ein Energietransport in umgekehrter Richtung statt. Der Rohrreaktor kann dabei entlang seiner Länge auf eine einheitliche Temperatur thermostatisiert sein. Alternativ können entlang der Länge des Rohrreaktors verschiedene Temperaturen eingestellt werden, z.B. eine höhere Temperatur in der Nähe des Reaktoreingangs und eine niedrigere Temperatur in der Nähe des Reaktorausgangs. Bisweilen kann es vorteilhaft sein, einen im Wesentlichen adiabatisch betriebenen Hauptreaktor mit einem im Wesentlichen isotherm betriebenen Nachreaktor zu kombinieren.

Das erfindungsgemäße Verfahren kann auch unter Verwendung einer Rührkesselkaskade durchgeführt werden.

Im erfindungsgemäßen Verfahren wird ein Verdünnungsmittel zur Lösung oder Dispergierung von Harnstoff und/oder Alkylgruppen-übertragendem Reagenz eingesetzt. Das Verdünnungsmittel wird vorzugsweise in solcher Menge eingesetzt, dass die entstehende Reaktionswärme bei im Wesentlichen adiabatischer Reaktionsführung zu einer Erhöhung der Temperatur des Reaktionsgemisches von höchstens 150°C, vorzugsweise höchstens 100°C, z. B. höchstens 80°C, insbesondere höchstens 60°C, führte

Das Verdünnungsmittel absorbiert einen großen Teil der entstehenden Reaktionswärme und begrenzt so den Temperaturanstieg beim Einsetzen der Reaktion. Zersetzungs- und Nebenreaktionen werden auf diese Weise begrenzt. Das Verdünnungsmittel wird vorzugsweise in einer Menge von 0,2 bis 1,0 mol pro mol reaktionsbegrenzender Komponente eingesetzt. Unter "reaktionsbegrenzende Komponente" wird diejenige Komponente, d.h. Harnstoff oder Alkylgruppen-übertragendes Reagenz, verstanden, die im stöchiometrischen Unterschuß eingesetzt wird, beziehungsweise eine beliebige der Komponenten, wenn diese in einem äquimolaren Verhältnis eingesetzt werden.

Das Verdünnungsmittel ist ein in flüssiger, z.B. öliger, Form vorliegendes O-Alkylisoharnstoff-Säureadditionssalz, das gleichzeitig als Harnstoff-Lösungsmittel dient.

Die Reaktionstemperatur beträgt beim erfindungsgemäßen Verfahren 40-200°C, vorzugsweise 60-120°C. Je nach Reaktionstemperatur wird dabei eine Verweilzeit von 5 Sekunden bis 10 Stunden, vorzugsweise 2 Minuten bis 1 Stunde, eingestellt. Es wurde überraschenderweise gefunden, dass man auch bei Temperaturen von 140°C und mehr arbeiten kann, wenn eine entsprechend kurz bemessene Reaktionszeit gewählt wird. Im Allgemeinen ist bevorzugt, dass die Verweilzeit t (in Minuten) und die Temperatur T (in K) folgender Gleichung genügen:

$$5 \leq \int_{\tau=0}^{\tau=t/\min} 2^{\frac{T(\tau)-363K}{10K}} \cdot d\tau \leq 60$$

worin $T(\tau)$ für die Temperatur steht, bei der das Reaktionsgemisch zum Zeitpunkt $\tau$ vorliegt.

Bei konstantem T vereinfacht sich die Gleichung zu:

$$5 \leq 2^{\frac{T-363K}{10K}} \cdot \frac{\Delta t}{\min} \leq 60$$

wenn für T eine mittlere Temperatur im Reaktor zugrunde gelegt wird, kann nach dieser vereinfachten Glei-

chung die Gesamtverweilzeit $\Delta t$ im Reaktor hinreichend genau abgeschätzt werden. Hiernach ergibt sich bei 363 K (90°C) eine Verweilzeit von 5 bis 60 min. Mit jeder Temperaturerhöhung um 10 K halbiert sich diese Verweilzeit, so dass bei 373 K (100°C) die Verweilzeit vorzugsweise 2,5 bis 30 min und bei 383 K (110°C) vorzugsweise 1,25 bis 15 min beträgt.

Der Harnstoff und/oder das Alkylgruppen-übertragende Reagenz können für sich oder gemeinsam gelöst oder suspendiert in dem Verdünnungsmittel in den Reaktor eingeführt werden. Es kann vorab ein Gemisch von Harnstoff und Alkylgruppen-übertragendem Reagenz, gegebenenfalls mit dem Verdünnungsmittel, hergestellt und in den Reaktor eingeführt werden. Zur Homogenisierung der Reaktionsmischung kann diese z.B. durch eine Homogenisiervorrichtung, wie einen statischen Mischer, geleitet werden.

Es wurde überraschenderweise gefunden, dass das O-Alkyl-isoharn-stoff-Säureadditionssalz hervorragende Lösungseigenschaften für Harnstoff besitzt. Das O-Alkylisoharnstoff-Säureadditionssalz ist außerdem ein geeignetes Reaktionsmedium für die erfindungsgemäße Umsetzung. Für den bevorzugten Fall, dass das gleiche O-Alkylisoharnstoff-Säureadditionssalz eingesetzt wird, wie nach dem erfindungsgemäßen Verfahren hergestellt wird, ist im Anschluß an die Umsetzung keine Entfernung des Lösungsmittels erforderlich. Zweckmäßigerweise handelt es sich bei dem zur Lösung des Harnstoffs zugeführten O-Alkylisoharnstoff-Säureadditionssalz um einen Teilstrom des am Reaktorausgang austretenden O-Alkylisoharnstoff-Säureadditionssalzes. Geeignete Rückführverhältnisse kann der Fachmann anhand einfacher Versuche leicht ermitteln. Als allgemeiner Rahmen läßt sich ein Rückführverhältnis von 1 bis 60%, vorzugsweise von 20 bis 40% angeben. Vorzugsweise wird neben dem O-Alkylisoharnstoff-Säureadditionssalz kein weiteres Lösungsmittel verwendet.

Das als Verdünnungs- und Lösungsmittel eingesetzte O-Alkyl-isoharnstoff-Säureadditionssalz wirkt als saurer Katalysator. Es ist im Allgemeinen bevorzugt, den sauren Katalysator dem Harnstoff beizumischen, bevor dieser in Kontakt mit dem Alkylgruppen-übertragenden Reagenz gebracht wird.

Als Alkylgruppen-übertragendes Reagenz können Alkylhalogenide der Formel $R^1$-X, wobei X für ein Halogen, insbesondere Cl, Br, I steht, eingesetzt werden. Besonders bevorzugt werden Dialkylsulfate der Formel $(R^1O)_2SO_2$ verwendet. Als Alkylhalogenide bzw. Dialkylsulfate kommen solche in Frage, bei denen $R^1$ verzweigte oder unverzweigte $C_1$-$C_{20}$-Alkylketten, vorzugsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl bedeuten kann.

Besonders bevorzugte Dialkylsulfate sind solche, bei denen $R^1$ für lineare oder verzweigte aliphatische Reste mit 1 bis 6 Kohlenstoffatomen, wie z.B. Methyl, Ethyl, Propyl, Butyl, Pentyl oder Hexyl, steht.

Das Molverhältnis von Harnstoff zu Alkylgruppen-übertragendem Reagenz liegt vorzugsweise zwischen 3:1 und 1:2, insbesondere 1,2:1 bis 1:1,2.

Das erfindungsgemäße Verfahren führt überraschenderweise zu einer hohen Selektivität der Alkylierung. Die Selektivität der O-Alkylierung liegt im Allgemeinen bei mehr als 85%, insbesondere mehr als 95%. Der Anteil an zwei- oder mehrfach alkyliertem Harnstoff liegt unter 2%, meistens unter 1%. Dies ist von besonderer Bedeutung, da diese Produkte bei der weiteren Umsetzung zu Nebenreaktionen führen können, die die Ausbeute und Reinheit der Folgeprodukte mindern können.

Das erfindungsgemäße Verfahren zeichnet sich außerdem dadurch aus, dass der Harnstoff und das Alkylgruppen-übertragende Reagenz in Form technischer Chemikalien, mit einer Reinheit von z.B. etwa 95-98 Gew.-% eingesetzt werden können.

Das erfindungsgemäß hergestellte O-Alkylisoharnstoff-Säureadditionssalz läßt sich ohne weitere Aufreinigung in einer zweiten Verfahrensstufe mit primären oder sekundären Aminen zu substituierten Guanidinverbindungen umsetzen. In bevorzugten Ausführungsformen erfolgt die Herstellung des O-Alkylisoharnstoff-Säureadditionssalzes, wie oben erörtert, lösungsmittelfrei und katalysatorfrei, so dass eine Abtrennung von Lösungsmittel oder Katalysator nach der Umsetzung nicht erforderlich ist. Die Umsetzung des Rohproduktes zum Guanidiniumsalz verläuft im Vergleich zur Umsetzung mit reinem O-Alkylharnstoff-Salz in vergleichbarer Ausbeute und führt zu einem Produkt vergleichbarer Reinheit. Ein weiterer Vorteil der direkten Umsetzung des rohen O-Alkylharnstoff-Säureadditionssalzes mit Aminen ist, dass im Rohprodukt noch vorhandene Reste an Alkylgruppen-übertragendem Reagenz beim Kontakt mit dem Amin quantitativ vernichtet werden. Dieser Schritt müßte bei der Herstellung von reinem O-Alkylharnstoff-Salz vor der weiteren Aufreinigung in einem eigenen Verfahrensschritt durchgeführt werden.

Geeignet für die Umsetzung mit den Isoharnstoffderivaten der Formel I sind aliphatische, cycloaliphatische oder aromatische primäre bzw. sekundäre Amine sowie Aminocarbonsäuren, Aminosulfonsäuren und Aminophosphonsäuren und deren Derivate. Weiterhin lassen sich auch primäre und sekundäre Amine umsetzen, die zusätzliche Amino- oder Iminogruppen enthalten, sowie aminogruppenhaltige Oligomere bzw. Polymere.

Als bevorzugt verwendete Amine sind alle die in Wasser oder in, mit Wasser mischbaren Lösungsmitteln löslichen, primären und sekundären Amine genannt. Bevorzugte Vertreter unter den einfachen Aminen sind u.a. Methylamin, Ethylamin, n-Propylamin, 2-Propylamin, Butylamin, Isobutylamin, Anilin, Benzylamin und Anthranilsäure. Weitere bevorzugt eingesetzte Aminogruppenhaltige Verbindungen sind u.a. Taurin und Aminocarbonsäuren wie Glycin, Alanin, Valin, Prolin, Leucin, Phenylalanin, Lysin, Methionin, Cystein, Asparaginsäure, Iminodiessigsäure, Sarkosin sowie deren Ester, Amide und Nitrile und deren Salze.

Ein besonders bevorzugtes Amin ist Sarkosin, das sowohl als freie Säure als auch insbesondere als Na- oder K-Salz in Form einer 5-60 Gew.-%igen, bevorzugt 35-45 Gew.-%igen wäßrigen Lösung verwendet werden kann.

In dem erfindungsgemäßen Verfahren können auch wasserlösliche, Aminogruppen enthaltende Oligomere und Polymere eingesetzt werden, wie Alkylendiamine, Dialkylentriamine bis hin zu Polyalkylenpolyamine oder Polyetherdiamine. Bevorzugte Vertreter dieser Gruppe sind Ethylendiamin, Propylendiamin, Butylendiamin, Hexamethylendiamin, Diethylentriamin, Triethylentetramin, Tetraethylenpentamin, Pentaethylenhexamin, Hexaethylenheptamin sowie verzweigte oder lineare Polyalkylenpolyamine.

Der Einsatz von technischen Produkten in dem erfindungsgemäßen Verfahren empfiehlt sich insbesondere dort, wo keine weiteren unerwünschten reaktiven Amine beigemischt sind und es aus wirtschaftlichen Gründen besonders vorteilhaft ist, weil beispielsweise die Aufreinigung des Amins teuer und aufwendig ist.

Die Umsetzung der substituierten Isoharnstoffderivate mit den oben genannten Aminen kann in Wasser oder einem mit Wasser mischbaren Lösungsmittel, oder einem Gemisch davon erfolgen. Üblicherweise wird dabei ein pH-Wert im Bereich des pK-Wertes des Amins verwendet, d.h. in einem pH-Bereich zwischen 6-14, bevorzugt zwischen 8,5-12,5, besonders bevorzugt im pH-Bereich von 9,5-12.

Das Molverhältnis von O-Alkylisoharnstoff-Säureadditionssalz zum primären oder sekundären Amin liegt pro reaktiver Aminogruppe im Bereich von 2:1 bis 1:2, vorzugsweise im Bereich von 1,5:1 bis 0,9:1. Bei Verwendung eines wertvollen Amins kann mit Vorteil das O-Alkylisoharnstoff-Säureadditionssalz im Überschuß eingesetzt werden.

Die Reaktionstemperaturen in der zweiten Stufe liegen im Bereich von -20°C bis 100°C, bevorzugt im Bereich von 0 bis 80°C, besonders bevorzugt zwischen 5 und 35°C.

Für die Reaktionsführung in der zweiten Verfahrensstufe spielt die Reihenfolge der Zugabe der Reaktanden keine besondere Bedeutung. In der Regel gibt man den substituierten Isoharnstoff zu dem primären oder sekundären Amin, das bevorzugt in wäßriger oder alkoholischer Lösung vorliegen kann.

Die Isolierung der gewünschten Guanidiniumderivate erfolgt in an sich bekannter Weise. So läßt sich in der Regel durch Abkühlen der Reaktionslösung auf -20 bis 60°C, insbesondere 0-40°C, das Wertprodukt kristallin erhalten. Nach Filtration kann gegebenenfalls durch eine weitere Umkristallisation die Reinheit verbessert werden. Es ist aber auch möglich, das Produkt mittels Extraktion aus dem Reaktionsgemisch zu entfernen, um es anschließend durch Destillation oder Kristallisation sauber zu isolieren.

Das folgende Beispiel veranschaulicht die erfindungsgemäßen Verfahren näher.

Beispiel

Synthese von O-Methylisoharnstoff-methylsulfat

5 Man führt kontinuierlich in einen Rohrreaktor (ID=0,4 cm, V=200 $cm^3$, Manteltemperatur=90°C, Verweilzeit=30 min) über eine Dosierpumpe eine Lösung von 110 g technischen Harnstoff in 178 g O-Methylisoharnstoff-methylsulfat pro Stunde zu. Über eine weitere Dosierpumpe mischt man diesem Stoffstrom 232 g technisches Dimethylsulfat pro Stunde zu. Man erhält pro Stunde 520 g eines dünnflüssigen Öles mit einem O-Methylisoharnstoff-methylsulfat-Gehalt laut HPLC von 86,7%, entsprechend 89,8% Ausbeute, von dem man 34% zum Lösen des Harnstoffs zurückführt. Der Gehalt an N-Methyl-O-Methylisoharnstoff-methylsulfat beträgt 0,53%, entsprechend einer Ausbeute von 0,5%; der Gehalt an Harnstoff beträgt 2,8%.

## Patentansprüche

**1.** Verfahren zur Herstellung von O-Alkylisoharnstoff der Formel I,

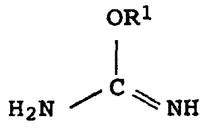

I

worin $R^1$ für $C_1$- bis $C_{20}$-Alkyl steht, in Form eines Säureadditionssalzes, bei dem Harnstoff und ein unter Alkylhalogeniden und Dialkylsulfaten ausgewähltes Alkylgruppen-übertragendes Reagenz, gelöst oder suspendiert in einem Verdünnungsmittel, in einem kontinuierlich betriebenen Reaktor bei einer Temperatur von 40-200°C umgesetzt werden, wobei das Verdünnungsmittel ein O-Alkylisoharnstoff-Säureadditionssalz ist.

2. Verfahren nach Anspruch 1, **dadurch gekennzeichnet, dass** das Verdünnungsmittel in solcher Menge eingesetzt wird, dass die entstehende Reaktionswärme bei im Wesentlichen adiabatischer Reaktionsführung zu einer Erhöhung der Temperatur des Reaktionsgemisches von höchstens 150°C führt.

3. Verfahren nach Anspruch 2, **dadurch gekennzeichnet, dass** die Verweilzeit t des Reaktionsgemisches im Reaktor (in Minuten) und die Temperatur T im Reaktor (in K) folgender Gleichung genügen

$$5 \leq \int_{\tau=0}^{\tau=t/min} 2^{\frac{T(\tau)-363K}{10K}} \cdot d\tau \leq 60$$

4. Verfahren nach einem der vorhergehenden Ansprüche, **dadurch gekennzeichnet, dass** das O-Alkylisoharnstoff-Säureadditionssalz vom Reaktorausgang teilweise zurückgeführt wird.

5. Verfahren nach einem der vorhergehenden Ansprüche, **dadurch gekennzeichnet, dass** das molare Verhältnis von Harnstoff zu Alkylgruppen-übertragendem Reagenz zwischen 3:1 und 1:2 liegt.

**Claims**

1. A process for the preparation of an O-alkylisourea of the formula I

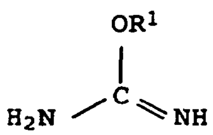

I

where $R^1$ is $C_1$- to $C_{20}$-alkyl, in the form of an acid addition salt, in which urea and an alkyl-transferring reagent selected from among alkyl halides and dialkyl sulfates, dissolved or suspended in a diluent, are reacted at 40-200°C in a continuously operated reactor, the diluent being an O-alkylisourea acid addition salt.

2. A process as claimed in claim 1, wherein the diluent is used in an amount such that the resulting heat of reaction in an essentially adiabatic reaction leads to an increase in the temperature of the reaction mixture of not more than 150°C.

3. A process as claimed in claim 2, wherein the residence time t of the reaction mixture in the reactor (in minutes) and the temperature T in the reactor (in K) satisfy the following equation

$$5 \leq \int_{\tau=0}^{\tau=t/min} 2^{\frac{T(\tau)-363K}{10K}} \cdot d\tau \leq 60$$

4. A process as claimed in any of the preceding claims, wherein some of the O-alkylisourea acid addition salt is recycled from the reactor exit.

**5.** A process as claimed in any of the preceding claims, wherein the molar ratio of urea to alkyl-transferring reagent is from 3:1 to 1:2.

## Revendications

**1.** Procédé pour la préparation d'O-alkylisourée de formule I,

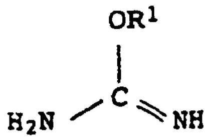

I

où $R^1$ désigne un alkyle en $C_1$ à $C_{20}$, sous forme d'un sel d'addition à un acide, dans lequel on fait réagir de l'urée et un réactif véhiculant des groupes alkyle choisi parmi les halogénures d'alkyle et les sulfates de dialkyle, dissous ou en suspension dans un agent de dilution, dans un réacteur opérant en continu, à une température de 40-200°C, tandis que l'agent de dilution est un sel d'addition à un acide de 0-alkylisourée.

**2.** Procédé selon la revendication 1, **caractérisé par le fait que** l'agent de dilution est introduit en une quantité telle que la chaleur de réaction formée dans la conduite de la réaction substantiellement adiabatique conduit à une élévation de la température du mélange réactionnel d'au plus 150°C.

**3.** Procédé selon la revendication 2, **caractérisé par le fait que** la durée de séjour t du mélange réactionnel dans le réacteur (en minutes) et la température T dans le réacteur (en °K) satisfont à la relation suivante

$$5 \leq \int_{\tau=0}^{\tau=t/min} 2^{\frac{T(\tau)-363K}{10K}} \cdot d\tau \leq 60$$

**4.** Procédé selon l'une des revendications précédentes, **caractérisé par le fait que** le sel d'addition à un acide de 0-alkylisourée provenant du réacteur est partiellement recyclé.

**5.** Procédé selon l'une des revendications précédentes, **caractérisé par le fait que** le rapport molaire de l'urée au réactif véhiculant des groupes alkyle se situe entre 3:1 et 1:2.